# EUROPEAN PATENT SPECIFICATION

(11) **EP 4 129 234 B1**
(45) Date of publication and mention of the grant of the patent: **15.04.2026**
(21) Application number: 21460032.2
(22) Date of filing: 03.08.2021
(51) Int. Cl.: A61F 2/14

(54) **METHOD OF FABRICATION OF AN OCULAR PROSTHESIS**
VERFAHREN ZUR HERSTELLUNG EINE AUGENPROTHESE
PROCÉDÉ DE FABRICATION D'UNE PROTHÈSE OCULAIRE

(43) Date of publication of application: 08.02.2023
(73) Proprietor: Optomed Piotr Jaworski, 41-500 Chorzów (PL)
(72) Inventor: Jaworski, Marcin, 41-500 Chorzów (PL); Jaworski, Piotr, 41-500 Chorzów (PL)
(74) Representative: Karczmitowicz, Teresa Ewa

(56) References cited:
- WO-A1-2019/124786
- GB-A- 2 589 698
- KR-B1- 102 120 610
- KR-B1- 102 284 794

## Description

The subject of the invention is a method of fabrication of an ocular prosthesis.

A method of forming an ocular prosthesis is disclosed in international patent application WO2021038243 A1. It comprises the following steps: receiving three dimensional scan data of a patient's eye, the three dimensional scan data including volumetric data of at least an iris region, a pupil region and a cornea region of the eye; and forming an ocular prosthesis by an additive manufacturing process. The ocular prosthesis has a body with a volume, wherein the body of the prosthesis includes a three dimensional (3D) iris region, a 3D pupil region and a 3D cornea region. The volumetric data determines the size and shape of the iris region, the pupil region and the cornea region of the prosthesis.

According to Korean patent KR102120610 B1 an exterior prosthetic eye manufacturing method comprises: a 3D scanning step of acquiring 3D scan data of a subject's face by using a photogrammetry scheme; a 3D modeling step of modeling male and female molds of an exterior prosthetic eye to be mounted around the subject's eye by using the 3D scan data acquired through the 3D scanning step; a 3D printing step of 3D-printing the male and female molds modeled through the 3D modeling step; a silicone molding step of injecting silicone into the male and female molds output in the 3D printing step to form an exterior prosthetic eye made of silicone; and a silicone coloring step of coloring, with a material including silicone, the exterior prosthetic eye molded in the silicone molding step.

An invention described in Chinese patent document CN109087387 A discloses an individualized 3D printing multifunctional artificial orbital implant and a preparation method thereof. The surface of the artificial orbital implant has a porous structure having circular pores. The porous structure is arranged in a gradient manner. The said prosthetic eye seat is provided with a special structure for fixing four extraocular muscles. The special structure consists of two elliptical holes intersecting and arranged close to each other. The extraocular muscles are attached to the elliptical holes, and they are preset with slits. After passing through the two elliptical holes, the thread is knotted and fixed at the mutual penetration point. The special structure is arranged at the four intersection points of the artificial eye seat at 30° north latitude and longitude 0°, 90°, 180° and 270°, so that the four extraocular muscles are fixed at the four intersection points. The prosthetic orbital implant is printed directly by the digital model of the prosthetic orbital implant using 3D printing technology. The artificial orbital implant has high matching degree with the healthy eye and reduces the risk of exposure and infection after implantation of the orbital implant. It has good mobility and good simulation effect with individualized ophthalmic prosthesis, and has good clinical application value.

Russian patent application RU2016135707 A1 discloses a method for making ocular prostheses in which linear dimensions and volume of an ocular cavity, as well as the structure of the walls surfaces of the ocular cavity, are pre-determined by digital scanning A 3D model of an ocular prosthesis is made by mechanical processing of a biologically compatible workpiece from acryl copolymers. Outer surface of prepared 3D model of the ocular prosthesis is coated with acrylic copolymer of the required tone corresponding to the eyeball sclera. Synthetic microstripes imitating sclera vessels are attached to the external surface by means of infrared radiation. Transparent lens with an iris is placed on the prepared rounded area on 3D model of the ocular prosthesis. A monolithic model is obtained by secondary coating of the volumetric model of the ocular prosthesis with transparent acrylic copolymer.

Chinese utility model application CN202128566 U discloses a composite nano-material ocular prosthesis that comprises a matrix, and a pupil as well as an iris pattern which is disposed on the middle part of the outer side surface of the matrix. A biological material (hydroxyapatite) coating compatible with tissues of human eyes is disposed on the inner side surface of the matrix. Thickness of the biomaterial coating is 0.5-1.0 mm. The ocular prosthesis sheet can be vascularized easily after being implanted into human eye socket. The composite nano-material ocular prosthesis is provided with a plurality of micropores communicated with each other, thereby vascular tissues around the part compatible with the tissues of human eyes can grow in the micropores. Synchronous rotation can be realized through the integration of fibrovascular tissues, and the ocular prosthesis looks more real. After the integration the ocular prosthesis can be cleaned without being taken out of the eye socket, thereby secondary infection during explanation and implantation of the ocular prosthesis can be prevented.

British patent application GB2589698 A describes a method of manufacturing an ocular prosthesis, and a prosthesis produced thereby. The method comprises: (i) scanning an anophthalmic or microphtalmic eye socket in order to measure a three dimensional (3D) fitting surface on which an ocular prosthetic is to be fitted, e.g. by optical coherence tomography, and (ii) manufacturing the prosthesis by an additive manufacturing process, e.g. polyjet printing. The prosthesis has a rear surface arranged to conform to the scanned fitting surface. The scanned information may be used to form a schematic of the prosthesis by which the manufacturing process is performed. The schematic may also include information on a colour, shape, size and pattern of the patient's sclera, pupil and iris, captured by imaging of the patient's eye. Usually, the shape of the rear surface of the ocular prosthesis is based on the impression of the anophthalmic socket or microphthalmic eye and surrounding tissue using an impression material. The rest of the shape of the prosthesis is determined based on standard parts. Both the iris and the sclera are hand painted whilst the patient is present in clinic to provide the desired appearance. According to the method described in GB2589698 A, forming an ocular prosthesis comprises: receiving 3D scan data of the patient's eye, the 3D scan data including volumetric data of at least an iris region, a pupil region and a cornea region of the eye; and forming an ocular prosthesis by an additive manufacturing process. The ocular prosthesis has a body with a volume, wherein the body of the prosthesis includes a 3D iris region, a 3D pupil region and a 3D cornea region, and the volumetric data determines the size and shape of the iris region, the pupil region and the cornea region of the prosthesis.

The subject matter of the present invention is a technological process that eliminates a need to produce expensive and time-consuming molds for casting and preparing individually manufactured prostheses, thus maximising reliable representation of the orbital geometry. The key step is to obtain a digital model of the prosthesis, including its color mapping for quick, easy and repeatable production, individualized for a specific patient. This process is advantageous due to shortening of manufacturing time of the prosthesis. Also, it eliminates time-consuming and expensive production of molds that are unstable and significantly reduce the number of prostheses made, and which take up a lot of storage space. Using them after a few years becomes impossible due to low durability of the mold and the dimensional changes with time.

Unless this leads to any misunderstandings, a prosthesis should be understood as any type of ocular prosthesis, both a prosthesis with orbital filling and epiprosthesis.

Modifications of ocular prosthesis according to the standard procedure require manual processing. Consequently, after adjusting the prosthesis, another mold should be made for later reconstruction of the prosthesis or alternatively, every time such modifications should be carried out manually. This virtually eliminates the process repeatability. On the other hand, in the described invention, modifications are made by computer modeling or the model is corrected manually, if it is easy to scan the prosthesis and make corrections to the digital prosthesis model for the subsequent manufacture of the product.

Change of the orbit shape, especially its shrinking, causes the necessity to change the shape or volume of the orbit filling over time. In the proposed solution, modifications can be made quickly without the need to repeat the entire technological process, it can also be done remotely, without the presence of the patient in the laboratory. The use of direct and indirect scanners allows for remote work - preparation of prostheses and epiprostheses in cooperating laboratories, without the need for physical presence of the patient. It is of great importance due to various unfavorable situations, e.g. limited patient mobility, and in the time of a pandemic. The invention is of great importance for patients living far away from prosthetic centers, facilitating or even enabling the proper supply of the patient with a prosthesis or epiprosthesis.

Taking a picture of the second eye and then transferring the graphics ensures repeatability and the most accurate reproduction of the color of the second eye in the prosthesis. This technology eliminates the tedious and inaccurate process of manually reproducing the color of the iris and sclera of the eye. Additionally, it does not require any artistic skills on the part of the operating personnel. This shortens the production process, and if there is a need to make another prosthesis, maximum repeatability is achieved. Due to the fact that the graphic data is stored in the database, there is no need to call the patient for an appointment to select the color and dimensions of the prosthesis. This reduces the production time, saves the patient's time and reduces the risk related to her/his mobility, such as during a pandemic.

The proposed method of producing an ocular prosthesis includes the following steps:
1. Making a geometric model of the eye socket through
   - direct orbital scanning with an orbital scanner or
   - indirect method by scanning an impression or a cast of the eye socket.
2. Modifying the obtained scan by manual or automatic modeling in the spatial modeling software.
3. Determining the geometric axis parallel to the axis of vision of the other eye or determining the axis in the case of making bi-ocular prostheses.
4. Producing a proper prosthesis from a biocompatible material through
   - simple 3D print or
   - composite 3D print for transparent elements (cornea or preparation of a place, so-called lodge, for their production in a separate technological process).
5. Applying graphics - the colours and patterns of the sclera, blood vessels and iris
   - in a prosthesis printing, transfer or painting process, preferably from a photograph of the other eye, or
   - application of iris graphics with a transparent element - a cornea imitating the anterior chamber of the eye.
6. Securing whole surface of the manufactured prosthesis or selected parts with biocompatible varnish.

Direct or indirect scanning enables the development of a digital model of the orbit and allows for repeatable fabrication of prostheses. So far, castings of the eye socket have been made, molds were created and then the prosthesis was manually adjusted in the technology of acrylic or silicone prostheses. On the other hand, in the technology of making glass prostheses, the shape of the eye socket is not reproduced at all, and the process is not repeatable. In this technology, a visit to the prosthesis manufacturer is indispensable. Moreover, inaccurate modeling of the prosthesis may cause pain and even contribute to the development of the socket syndrome, making it impossible to fit any prosthesis after some time.

Direct or indirect scanning, combined with a photograph of the eye or the use of a color picker to select the colors of the iris, sclera and its blood vessels, allows for remote work without a need of the patient's visit in the prosthesis laboratory. Such a possibility is not offered by any currently used technology of the prosthesis production.

The shape of the prosthesis according to the invention can be modified based on a digital model. The modified shape is fully reproducible. However, in the technology of acrylic or silicone prostheses, after modification, another form should be made or each time modifications should be made manually, which eliminates the repeatability of production. On the other hand, the modification of the glass prosthesis is possible only in the process of its production, because after it solidifies, the glass becomes too brittle for corrective treatment. If a correction is required, a new prosthesis should be made.

The claimed invention offers the possibility of color matching by applying other modified graphics. Moreover, and important in the manufacturing process, multiple prosthesis models can be prepared at the same time. Additionally, the proposed technology allows the application of a translucent cornea in the form of a button on the iris graphic. This allows for depth and simulates the natural shape of the anterior segment of the eyeball - the anterior chamber. Changing the color of the iris or sclera prostheses in the production of acrylic, silicone or glass prostheses requires the prosthesis to be remade. The use of the mold allows for the production of only one prosthesis and in case of its damage in the production process, the process must be repeated.

In more sophisticated variant of the invention an additional layer of nanoparticles (nanolayer) is applied on the surface of peripheral part of the prosthesis, i.e. in vicinity of its edge. The areas selected for covering with the nanolayer must be free of protective varnish. The tissue of the eye socket may perform stronger adhesion to the prosthesis and enable its more natural performance, including susceptibility to slight movements controlled by the ocular muscles.

An embodiment of the epiprosthesis made with the method according to the invention is shown in the drawing in which:

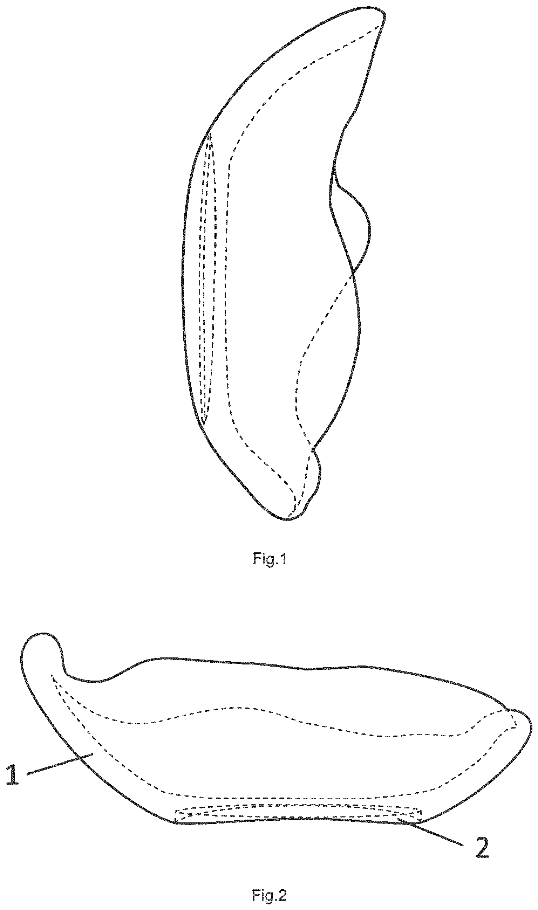

Fig. 1 shows perspective view of classical single-part epiprosthesis;
Fig. 2 is a side view of the epiprosthesis (1) with shaped lodge (2) for a cornea button;

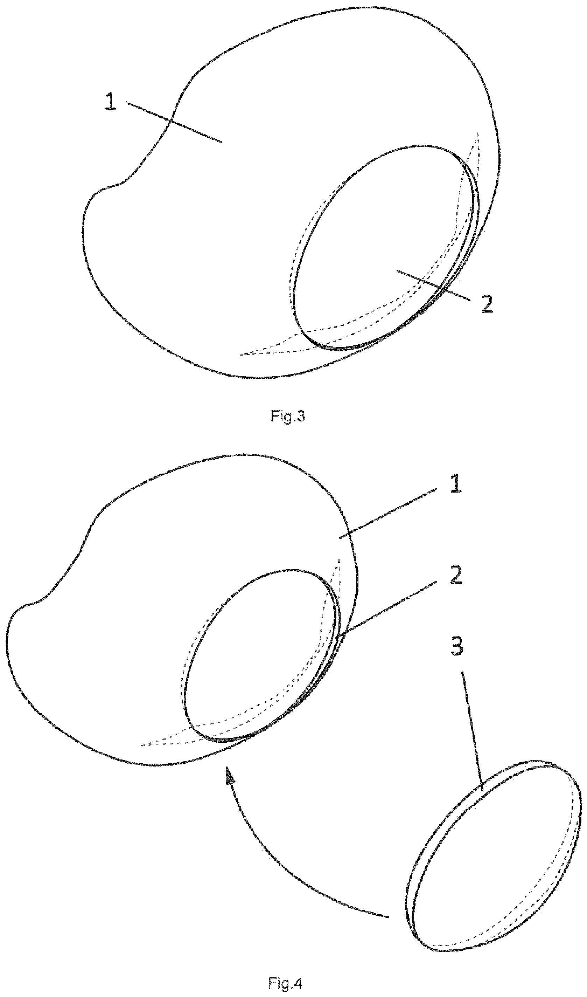

Fig. 3 shows the epiprosthesis from Fig. 2 in front-perspective view;
Fig. 4 illustrates the epiprosthesis from Fig. 3 and the cornea button (3);

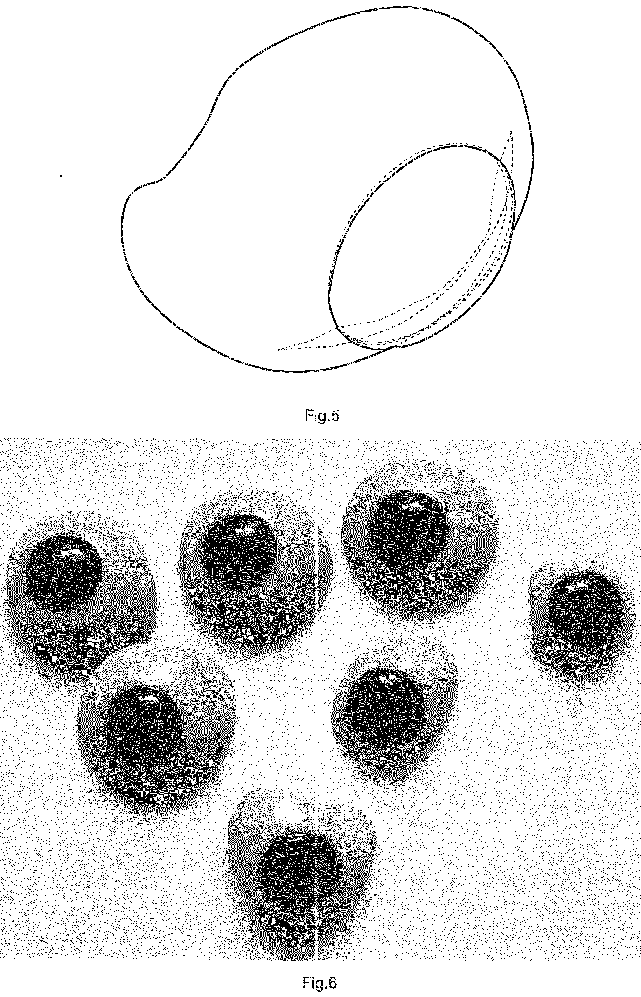

Fig. 5 shows final two-part epiprosthesis;
Fig. 6 shows photographs of various embodiments of the ocular prostheses fabricated with use of claimed method, and

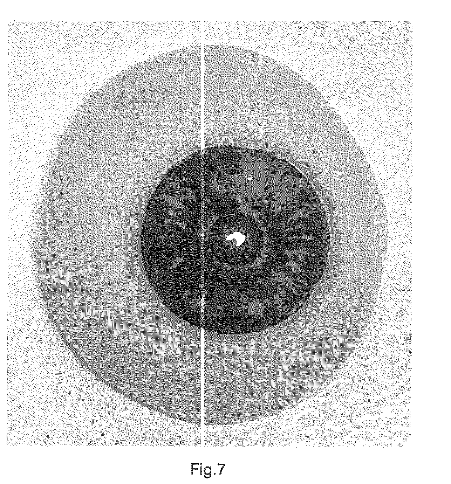

Fig. 7 shows a close-up view of the epiprosthesis with visible light-colour rim around the iris mimicking the corneal limbus.

In the embodiment of the prosthesis obtained by the method according to the invention, an orbital impression was made with the use of Vinyl PolySiloxane (VPS). A photo-curable resin with high biocompatibility level (ISO 10993 and/or USP Class VI Medical Grade) was used as the material for additive printing (3D) of the prosthesis. The composition of the resin is given in the table, where a solvent content counts for 100%. Material properties after curing: Shore Hardness 75-90D (ISO 178 measurement method), Elongation at break 2.0-5.0% (ISO D638M measurement method), Flexural modulus 1800-2400 MPa (ISO 20795-1 measurement method). The finished prosthesis was covered with a hydrophobic photo-curable varnish (UV) using an airbrush under a pressure of 0.5-1.0 Bar. The varnish contains methyl methacrylate (25-50%), diphenyl (2,4,6-trimethylbenzoyl) phosphines (3-5%) and a solvent up to 100%. A UV lamp (365 nm and/or 405 nm) was used for the photo-curing, irradiating the prosthesis on each side for 20 s.

| **Chemical Name** | **Trade names / Synonyms** | **CAS No.** | **Content (%)** |
|---|---|---|---|
| α,α'-[(1-Methylethylidene)di-4,1-phenylene]bis[ω-[(2-methyl-1-oxo-2-propenyl)oxy]poly(oxy- 1,2-ethanediyl) | - | 41637-38-1 | 20~35 |
| 7,7,9(or 7,9,9)-Trimethyl-4,13-di oxo-3,14-dioxa-5,12- diazahexadecane-1,16-diyl 2-methyl-2-propenoate | - | 72869-86-4 | 20~28 |
| 2-Methyl-2-propenoic acid 1,2-ethanediylbis(oxy-2,1- ethanediyl) ester | 2,2'-Ethylenedioxy diethyl dimethacrylate | 109-16-0 | 20~25 |
| Phenylbis(2,4,6-trimethylbenzoyl) phosphine oxide | Methanone, 1,1'-(phenylphosphinylide ne)bis[1- (2,4,6-trimethylphenyl)- | 162881-26-7 | 1~10 |

A nanolayer applied on a circumference of the ocular prosthesis may consist of 60% of hydroxyapatite and 40% of beta-TCP (tricalcium phosphate). The obtained nanolayer has macropores of a diameter comprised in a range of 200-800 micrometers, and also has micropores of a diameter comprised in a range of 1-10 micrometers.

An additive of nanosilver known for its antibacterial properties may be included in the nanolayer. Nanosilver particles may be applied on the surface of nanolayer, or may be incorporated into the nanolayer

The nanolayer should not be applied on the varnish layer, neither the varnish on the nanolayer surface.

## Claims

1. A method of fabrication of an ocular prosthesis including the following steps:
a. making a geometric model of the eye socket through direct orbital scanning with an orbital scanner or indirectly by scanning an impression or a cast of the eye socket;
b. modifying of the obtained scan by manual or automatic modeling with use of a spatial modeling software;
c. determining the geometric axis parallel to the axis of vision of the other eye or determining the axis in the case of making bi-ocular prostheses;
d. completing a digital model of the prosthesis with use of geometric model of the eye, its modifications made with use of a spatial modeling software, and geometric axis;
e. making a proper prosthesis from a biocompatible material with use of 3D printing according to the digital model of the prosthesis;
f. smoothing and finishing the surface of the prosthesis;
g. applying graphics comprising the colours and patterns of the sclera, blood vessels and iris on the prosthesis;
h. securing the surface of the manufactured prosthesis (1) or a part thereof with a biocompatible varnish.

2. The method according to claim 1, wherein 3D printing is a one-step process of additive manufacturing of the whole prosthesis.

3. The method according to claim 1, wherein 3D printing is a multi-step process of additive manufacturing comprising the steps of printing of the prosthesis main body, and further printing of a transparent part mimicking the cornea on the prosthesis main body.

4. The method according to claim 1, wherein 3D printing is a process of composite additive manufacturing comprising the steps of printing of the prosthesis main body with a lodge for a transparent button mimicking the anterior chamber and the cornea which is made separately, and joining both parts together.

5. The method according to any of claims 1 to 4, wherein graphics of the sclera, blood vessels and iris is applied in a prosthesis printing, transfer or painting process, preferably from a photograph of the other eye.

6. The method according to claim 4, wherein graphics of the iris is applied on the bottom of the lodge (2) or on the back side of the transparent button (3).

7. The method according to any of claims 1 to 6, wherein graphics of the sclera and iris is included in the digital model of the prosthesis.

8. The method according to any of claims 1 to 7, wherein it includes a stage of 3D-printing of separate ocular filling which is further fixed to the prosthesis.

9. The method according to any of claims 1 to 8, wherein the surface of peripheral edges of the prosthesis is left free from the biocompatible varnish.

10. The method according to claim 9, wherein peripheral edges of the prosthesis are additionally covered with a biocompatible nanolayer.

11. The method according to claim 10, wherein the nanolayer consists of 60% of hydroxyapatite and 40% of beta-TCP.

12. The method according to claim 10 or 11, wherein the nanolayer has macropores of a diameter comprised in a range of 200-800 micrometers, and also has micropores of a diameter comprised in a range of 1-10 micrometers.

13. The method according to any of claims 10 to 12, wherein nanosilver particles are applied on peripheral edges of the prosthesis.

14. The method according to claim 13, wherein the nanosilver particles are introduced into the nanolayer.

## Patentansprüche

1. Verfahren zur Herstellung einer Augenprothese, das die folgenden Schritte umfasst:
a. Erstellen eines geometrischen Modells der Augenhöhle durch direktes Scannen der Augenhöhle mit einem Augenhöhlen-Scanner oder indirekt durch Scannen eines Abdrucks oder eines Abgusses der Augenhöhle;
b. Modifizieren des erhaltenen Scans durch manuelles oder automatisches Modellieren unter Verwendung einer räumlichen Modellierungssoftware;
c. Bestimmen der geometrischen Achse parallel zur Sehachse des anderen Auges oder Bestimmen der Achse im Falle der Herstellung von binokularen Prothesen;
d. Erstellen eines digitalen Modells der Prothese unter Verwendung des geometrischen Modells des Auges, der mittels einer räumlichen Modellierungssoftware vorgenommenen Modifikationen und der geometrischen Achse;
e. Herstellen einer geeigneten Prothese aus einem biokompatiblen Material mittels 3D-Druck gemäß dem digitalen Modell der Prothese;
f. Glätten und Endbearbeiten der Oberfläche der Prothese;
g. Aufbringen von Grafiken mit den Farben und Mustern der Sklera, der Blutgefäße und der Iris auf die Prothese;
h. Versiegeln der Oberfläche der hergestellten Prothese (1) oder eines Teils davon mit einem biokompatiblen Lack.

2. Verfahren nach Anspruch 1, wobei der 3D-Druck ein einstufiger Prozess der additiven Fertigung der gesamten Prothese ist.

3. Verfahren nach Anspruch 1, wobei der 3D-Druck ein mehrstufiger Prozess der additiven Fertigung ist, der die Schritte des Druckens des Hauptkörpers der Prothese und des anschließenden Druckens eines transparenten Teils, der die Hornhaut auf dem Hauptkörper der Prothese nachahmt, umfasst.

4. Verfahren nach Anspruch 1, wobei der 3D-Druck ein Verfahren der additiven Verbundfertigung ist, das die Schritte des Druckens des Hauptkörpers der Prothese mit einer Aufnahme für einen transparenten Knopf, der die Vorderkammer und die Hornhaut nachahmt und separat hergestellt wird, sowie des Zusammenfügens beider Teile umfasst.

5. Verfahren nach einem der Ansprüche 1 bis 4, wobei Grafiken der Sklera, der Blutgefäße und der Iris in einem Druck-, Transfer- oder Malprozess auf die Prothese aufgebracht werden, vorzugsweise auf der Grundlage einer Fotografie des anderen Auges.

6. Verfahren nach Anspruch 4, wobei Grafiken der Iris auf der Unterseite der Aufnahme (2) oder auf der Rückseite des transparenten Knopfes (3) aufgebracht werden.

7. Verfahren nach einem der Ansprüche 1 bis 6, wobei Grafiken der Sklera und der Iris in das digitale Modell der Prothese aufgenommen sind.

8. Verfahren nach einem der Ansprüche 1 bis 7, das einen Schritt des 3D-Drucks eines separaten okulären Füllkörpers umfasst, der anschließend an der Prothese befestigt wird.

9. Verfahren nach einem der Ansprüche 1 bis 8, wobei die Oberfläche der peripheren Ränder der Prothese frei von dem biokompatiblen Lack bleibt.

10. Verfahren nach Anspruch 9, wobei die peripheren Ränder der Prothese zusätzlich mit einer biokompatiblen Nanobeschichtung überzogen werden.

11. Verfahren nach Anspruch 10, wobei die Nanobeschichtung zu 60 % aus Hydroxylapatit und zu 40 % aus Beta-TCP besteht.

12. Verfahren nach Anspruch 10 oder 11, wobei die Nanobeschichtung Makroporen mit einem Durchmesser im Bereich von 200 bis 800 Mikrometern sowie Mikroporen mit einem Durchmesser im Bereich von 1 bis 10 Mikrometern aufweist.

13. Verfahren nach einem der Ansprüche 10 bis 12, wobei Nanosilberpartikel auf die peripheren Ränder der Prothese aufgebracht werden.

14. Verfahren nach Anspruch 13, wobei die Nanosilberpartikel in die Nanobeschichtung eingebracht werden.

## Revendications

1. Procédé de fabrication d'une prothèse oculaire comprenant les étapes suivantes :
a. réalisation d'un modèle géométrique de l'orbite oculaire par scannage direct de l'orbite au moyen d'un scanner orbital ou, indirectement, par scannage d'une empreinte ou d'un moulage de l'orbite oculaire ;
b. modification du scan obtenu par modélisation manuelle ou automatique à l'aide d'un logiciel de modélisation spatiale ;
c. détermination de l'axe géométrique parallèle à l'axe de vision de l'autre œil ou détermination de l'axe dans le cas de la fabrication de prothèses binoculaires ;
d. réalisation d'un modèle numérique de la prothèse à l'aide du modèle géométrique de l'oeil, de ses modifications effectuées à l'aide d'un logiciel de modélisation spatiale et de l'axe géométrique ;
e. fabrication d'une prothèse appropriée à partir d'un matériau biocompatible à l'aide d'une impression 3D selon le modèle numérique de la prothèse ;
f. lissage et finition de la surface de la prothèse ;
g. application de graphismes comprenant les couleurs et les motifs de la sclère, des vaisseaux sanguins et de l'iris sur la prothèse ;
h. protection de la surface de la prothèse fabriquée (1) ou d'une partie de celle-ci au moyen d'un vernis biocompatible.

2. Procédé selon la revendication 1, dans lequel l'impression 3D est un processus en une seule étape de fabrication additive de l'ensemble de la prothèse.

3. Procédé selon la revendication 1, dans lequel l'impression 3D est un processus de fabrication additive en plusieurs étapes comprenant les étapes d'impression du corps principal de la prothèse, puis d'impression d'une partie transparente imitant la cornée sur le corps principal de la prothèse.

4. Procédé selon la revendication 1, dans lequel l'impression 3D est un processus de fabrication additive composite comprenant les étapes d'impression du corps principal de la prothèse avec un logement pour un bouton transparent imitant la chambre antérieure et la cornée, ledit bouton étant fabriqué séparément, et d'assemblage des deux parties.

5. Procédé selon l'une quelconque des revendications 1 à 4, dans lequel des graphismes de la sclère, des vaisseaux sanguins et de l'iris sont appliqués au cours d'un procédé d'impression, de transfert ou de peinture de la prothèse, de préférence à partir d'une photographie de l'autre œil.

6. Procédé selon la revendication 4, dans lequel les graphismes de l'iris sont appliqués sur le fond du logement (2) ou sur la face arrière du bouton transparent (3).

7. Procédé selon l'une quelconque des revendications 1 à 6, dans lequel les graphismes de la sclère et de l'iris sont inclus dans le modèle numérique de la prothèse.

8. Procédé selon l'une quelconque des revendications 1 à 7, dans lequel il comprend une étape d'impression 3D d'un remplissage oculaire séparé, lequel est ensuite fixé à la prothèse.

9. Procédé selon l'une quelconque des revendications 1 à 8, dans lequel la surface des bords périphériques de la prothèse est laissée libre de tout vernis biocompatible.

10. Procédé selon la revendication 9, dans lequel les bords périphériques de la prothèse sont en outre recouverts d'une nanocouche biocompatible.

11. Procédé selon la revendication 10, dans lequel la nanocouche est composée à 60 % d'hydroxyapatite et à 40 % de bêta-TCP.

12. Procédé selon la revendication 10 ou 11, dans lequel la nanocouche présente des macropores dont le diamètre est compris entre 200 et 800 micromètres, et comporte également des micropores dont le diamètre est compris entre 1 et 10 micromètres.

13. Procédé selon l'une quelconque des revendications 10 à 12, dans lequel des particules de nano-argent sont appliquées sur les bords périphériques de la prothèse.

14. Procédé selon la revendication 13, dans lequel les particules de nano-argent sont introduites dans la nanocouche.
